(19) Europäisches Patentamt
European Patent Office
Office européen des brevets

(11) EP 2 272 521 B1

(12) EUROPEAN PATENT SPECIFICATION

(45) Date of publication and mention of the grant of the patent:
**20.02.2013 Bulletin 2013/08**

(21) Application number: **09726917.9**

(22) Date of filing: **25.03.2009**

(51) Int Cl.:
***A61K 31/7016*** *(2006.01)* ***A61P 3/00*** *(2006.01)*
***A61P 3/04*** *(2006.01)* ***A61P 3/10*** *(2006.01)*

(86) International application number:
**PCT/JP2009/055978**

(87) International publication number:
**WO 2009/122983 (08.10.2009 Gazette 2009/41)**

# (54) ISOMALTULOSE FOR THE TREATMENT OF KETOSIS CASUED BY EXERCISING

ISOMALTULOSE ZUR BEHANDLUNG VON DURCH BEWEGUNG VERURSACHTER KETOSE

ISOMALTULOSE DESTINÉE AU TRAITEMENT DE LA CÉTOSE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **31.03.2008 JP 2008093186**

(43) Date of publication of application:
**12.01.2011 Bulletin 2011/02**

(73) Proprietor: **Mitsui Sugar Co., Ltd.**
**Chuo-ku**
**Tokyo 103-8423 (JP)**

(72) Inventors:
- **OKUNO, Masahiro**
  **Chigasaki-shi**
  **Kanagawa 253-0042 (JP)**
- **SUZUKI, Koji**
  **Tokyo 103-8423 (JP)**

(74) Representative: **Grünecker, Kinkeldey, Stockmair & Schwanhäusser**
**Leopoldstrasse 4**
**80802 München (DE)**

(56) References cited:
**WO-A1-2009/137838**

- **DATABASE MEDLINE [Online] US NATIONAL LIBRARY OF MEDICINE (NLM), BETHESDA, MD, US; October 2002 (2002-10), LINA B A R ET AL: "Isomaltulose (Palatinose): a review of biological and toxicological studies.", XP002667382, Database accession no. NLM12387299 & FOOD AND CHEMICAL TOXICOLOGY : AN INTERNATIONAL JOURNAL PUBLISHED FOR THE BRITISH INDUSTRIAL BIOLOGICAL RESEARCH ASSOCIATION OCT 2002 LNKD-PUBMED:12387299, vol. 40, no. 10, October 2002 (2002-10), pages 1375-1381, ISSN: 0278-6915**
- **ACHTEN JUUL ET AL.: 'Exogenous Oxidation of Isomaltulose Is Lower than That of Sucrose during Exercise in Men' JOURNAL OF NUTRITION vol. 137, no. 5, May 2007, pages 1143 - 1148, XP008143433**
- **KOTARO EDA: 'Shin Seihin Kaihatsu no Tameno Kanmiryo · Tenkabutsu · Shin Sozai Palatinose no Tokusei Oyobi Riyo' BEVERAGE JAPAN vol. 22, no. 11, 20 November 1999, pages 50 - 53, XP008145953**
- **J. H. KOESLAG ET AL.: 'The effects of alanine, glucose and starch ingestion on the ketosis produced by exercise and by starvation.' THE JOURNAL OF PHYSIOLOGY vol. 325, April 1982, pages 363 - 376, XP008143435**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to isomaltulose, a ketone body accumulation inhibitor, which inhibits the accumulation of ketone bodies caused by exercising in diabetic patients, patients with metabolic syndrome, and obese persons.

### Background Art

Ketosis represents the state in which ketone bodies are increased in the body, and it is caused by the fact that lipid metabolism, instead of carbohydrate metabolism, is promoted to produce excessive ketone bodies by diabetes, fasting, exercising and the like. Since ketone bodies are acidic, progression of the ketosis causes blood to shift to acid (ketoacidosis) and causes disordered consciousness or severe coma in the serious cases, and can cause death.

A mechanism of ketone body production is considered as follows. Fatty acids released from neutral fat turn into acyl-CoAs in the liver. The acyl-CoAs combine with glycerol to resynthesize neutral fat when insulin is sufficient, but on the other hand, they are β-oxidized into acetyl-CoA when the insulin is deficient. When the acetyl-CoA is excessive, or gluconeogenesis is promoted by the shortage of carbohydrates in the body by fasting, diabetes and the like, ketone bodies are produced from the acetyl-CoA (Non-Patent Documents 1 and 2). Ketone bodies tend to increase in persons who do not usually exercise, while they do not tend to increase in persons who usually work out (Non-Patent Document 2).

Administration of insulin or sugar such as glucose is generally conducted for prevention and treatment of ketosis. However, since diabetic patients fall into the state of high blood sugar levels when they take sugars such as glucose, sugar intake is restricted for them. Though diabetic patients need to improve their physical conditions by exercising, they may have difficulties in exercising without anxieties due to the risk of high blood sugar levels, ketosis and acidosis. Also, when patients with metabolic syndrome and obese persons take sugars such as glucose to prevent accumulation of ketone bodies caused by high-intensity exercising, oxidative metabolism of lipids by exercising is inhibited, and hence they may not get sufficient exercising effects.

On the other hand, fructose intake causes little elevation of blood sugar levels, but it is not suitable to supply muscles with energy because a large amount of fructose intake is metabolized to lipids in the liver (Non-Patent Document 3).

A ketone body urinary excretion accelerator containing diglyceride as an active ingredient is known as an eliminant for ketone derivative into urine (Patent Document 1).

Patent Document 1: Japanese Patent Application Laid-Open Publication No. 8-26988
Non-Patent Document 1: Examination and Technology Vo1.32, No.3, pp276-277,2004
Non-Patent Document 2: Japanese Journal of Biomechanics in Sports and Exercise Vol.1, No.1, pp105-119, 1997
Non-Patent Document 3: Nutrition Reviews Vol.63, No.5, pp133-157, 2005

### Disclosure of the Invention

#### Technical Problem

In such a situation, for subjects such as diabetic patients, patients with metabolic syndrome, and obese persons who have an accumulation of ketone bodies caused by exercising, an agent to prevent accumulation of ketone bodies caused by exercising and to allow them to get sufficient exercising effects is required. Hence, the object of the present invention is to provide isomaltulose for use in the treatment of ketosis caused by exercising in diabetic patients, patients with metabolic syndrome, and obese persons.

#### Solution to Problem

The above problem is solved by the compound for use according to claim 1.

The isomaltulose for use according to the present invention can inhibit accumulation of ketone bodies caused by exercising in subjects such as diabetic patients, patients with metabolic syndrome, and obese persons who have an accumulation of ketone bodies caused by exercising. Moreover, it can inhibit accumulation of ketone bodies, maintaining oxidative metabolism of lipids caused by exercising to maintain sufficient exercising effects.

## Brief Description of the Drawings

Figure 1 is a graph showing blood glucose levels changes over time by drink intake after exercising;
Figure 2 is a graph showing blood insulin levels changes over time by drink intake after exercising;
Figure 3 is a graph showing blood C-peptide levels changes over time by drink intake after exercising;
Figure 4 is a graph showing blood free fatty acid levels changes over time by drink intake after exercising;
Figure 5 is a graph showing blood lactic acid levels changes over time by drink intake after exercising;
Figure 6 is a graph showing respiratory quotient changes over time by drink intake after exercising;
Figure 7 is a graph showing the amount of oxidized carbohydrate changes over time by drink intake after exercising;
Figure 8 is a graph showing the amount of oxidized fat changes over time by drink intake after exercising;
Figure 9 is a graph showing blood neutral fatty acid levels changes over time by drink intake after exercising;
Figure 10 is a graph showing blood epinephrine levels changes over time by drink intake after exercising;
Figure 11 is a graph showing blood nolepinephrine levels changes over time by drink intake after exercising;
Figure 12 is a graph showing blood 3-OHBA levels changes over time by drink intake after exercising; and
Figure 13 is a graph showing blood total ketone bodies levels changes over time by drink intake after exercising.

## Best Modes for Carrying Out the Invention

The preferred embodiments of the present invention will be described in detail below.

In the present invention, "isomaltulose" is also referred to as palatinose and it is a disaccharide formed by an $\alpha$-1,6-glucosyl bond between glucose and fructose. "Palatinose" is a registered trademark by Mitsui Sugar Co., Ltd.

Palatinose may be in the form of hydrate. Palatinose monohydrate has melting point of 123°C to 124°C, specific optical rotation of $[\alpha]^{20}_{D}$ = +97.0-99.0° (C = 0.04), Fehling solution reducing ability of 52% of glucose and solubility into 100 g water of 38.4 g at 20°C. Moreover, sweetness quality of its aqueous solution is good and its degree of sweetness is about 40% of sucrose.

Palatinose is found in honey in nature. It also exists among transfer products produced by the action of $\alpha$-glucosyltransferase (isomaltulose synthase) derived from bacteria and yeast on sucrose.

In industrial production, palatinose is produced by effects of $\alpha$-glucosyltransferase derived from bacteria such as Protaminobacter rubrum or Serratia plymuthica on sucrose.

In the present invention palatinose is used for preventing accumulation of ketone bodies in subjects who produce and accumulate ketone bodies in the body by exercising. The subjects include humans who accumulate ketone bodies by exercising, particularly, diabetic patients, patients with metabolic syndrome and obese persons, and subjects suspected thereof. Patients with metabolic syndrome are for example, subjects who fit "definition and diagnostic criteria of metabolic syndrome" announced at the 102nd meeting of the Japanese Society of Internal Medicine (held in April 2005). Obese persons are for example, subjects who have BMIs of 25.0 or more. The criteria described above are original Japanese criteria, but the present invention is not limited to Japanese.

The term "ketone body" is a generic term for acetone, acetoacetic acid and 3-hydroxybutyric acid (3-OHBA). Acetoacetic acid is synthesized from acetyl-CoA as the material. Acetoacetic acid is reduced to 3-hydroxybutyric acid, followed by decarboxylation to produce acetone. Acetoacetic acid and 3-hydroxybutyric acid are present in blood. When the use of sugar is insufficient (severe diabetes, starvation, a shortage of sugar intake and the like), blood ketone body levels increase, and a part of the ketone bodies are excreted in expiration or urine. The amounts of produced and accumulated ketone bodies in the body can be examined by measuring blood ketone bodies, ketone bodies in urine and/or acetone in expiration. As these measuring methods, methods known in the art can be used. Ketone bodies in the body are normally negative but they may turn to be positive by high-intensity exercising. Also, while in case of ketone bodies being detected at ordinary times in diabetic patients, they may be under poorly controlled conditions.

The isomaltulose for use according to the present invention can be used alone or in combination with other ingredients. Other ingredients include known pharmaceutically acceptable excipients and carriers and in addition, they may include sucrose, flour, starch, dextrine, high-fructose corn syrup (HFCS) and the like. In the mixtures described above, the weight ratio of palatinose is preferably 99.99% to 10%, and more preferably, 99.99% to 20%.

A mixture of palatinose and other carbohydrates which are nonreducing sugars may be used. This reduces causes of coloration so that food may be less colored. Further, platinose may be combined with other carbohydrates with high solubility. This leads to less crystallization.

Palatinose may be used in the combination with sweeteners such as sucrose and high-fructose corn syrup. This decreases sweetness of sucrose, glucose and high-fructose corn syrup to produce food products with light and low sweetness.

Also, crystalline palatinose, powdered palatinose, palatinose syrup and/or trehalulose syrup can be used.

Crystalline palatinose (Crystalline Palatinose-IC, trade name, Mitsui Sugar Co., Ltd.) and powdered palatinose (Palatinose Powder-ICP, trade name, Mitsui Sugar Co., Ltd.) include 99.0% or more of palatinose (including crystalline water). Palatinose syrups (Palatinose Syrup-ISN and Palatinose Syrup-TN, trade name, Mitsui Sugar Co., Ltd.) include 11% to 17% of palatinose and 53% to 59% of trehalulose. Trehalulose syrup (Mildear-75 and Mildear-85, trade name, Mitsui Sugar Co., Ltd.) includes 8% to 13% of palatinose and 83% to 89% of trehalulose.

The form of the isomaltulose for use according to the present invention described above is not particularly limited and it includes a fondant, granule, tablet, syrup, ampuled liquid medicine, refreshing drink, jelly drink and powder drink. Moreover, the isomaltulose for use according to the present invention can be combined with materials which are usable for food, quasi-drug and medicine, and processed to food for specialized health uses, functional food, health food, quasi drug or medicine.

The isomaltulose for use according to the present invention is preferably taken after exercising. In taking palatinose, a fondant, granule, tablet or, syrup containing palatinose as an active ingredient may be taken as it is, and it is preferably taken with water, or dissolved or suspended in water to be taken as an ampuled liquid medicine or refreshing drink. The quantity of water to dissolve palatinose can be properly decided and 25 ml to 1000 ml of water is preferably used per 10 g of a mixture containing palatinose as an active ingredient and more preferably, 40 ml to 500 ml is used. Palatinose intake is preferably 0.5 g or more per kg of body weight, more preferably 0.7 g or more, and still more preferably 1 g or more. The intake method of the ketone body accumulation inhibitor of the present invention includes oral intake.

## Examples

The preferred examples of the present invention will be described in detail below.

Example 1: drinking test

To male subjects with suspected metabolic syndrome and 85 cm or more of abdominal circumferences who lack in exercise, the following test was conducted. Table 1 shows the physical measurements and metabolic profiles of the subjects. The subjects, who had family histories of cardiopathy or type 2 diabetes and/or had dyslipidemia, were eliminated from the test. Palatinose or high-fructose corn syrup was dissolved in 500 ml of water so that its intake per kg of body weight could be 1.0 g of the solid, and sucralose, a high intensity sweetener, was dissolved in 500 ml of water so that its intake per kg of body weight could be 1.33 mg of the solid that gives the degree of sweetness between the high-fructose corn syrup and palatinose solutions, to prepare three kinds of test drinks. For these three kinds of drinks, drinking tests were conducted, respectively and each test was conducted at intervals of ten days or longer. The high-fructose corn syrup is composed of glucose and fructose, the same constituent sugars as sucrose and palatinose, and its calorie per the solid is 4 kcal/g which is the same value as that of sucrose and palatinose.

[Table 1]

| Profile | Value | |
|---|---|---|
| Age (years old) | 46.0 ±2.3 | (38-59) |
| Body Height (cm) | 171.2 ±1.9 | (161.1-182.5) |
| Body Weight (kg) | 79.4 ±2.8 | (72.1-100.2) |
| BMI (kg/$m^2$) | 27.1 ±1.0 | (23.1-32.7) |
| Abdominal Circumference (cm) | 95.9 ±1.4 | (91.8-102.6) |
| Body Fat (%) | 25.7 ±1.2 | (22.8-30.6) |
| $VO_2$max (ml/kg/min) | 32.0 ±1.2 | (25.7-35.9) |
| 50% $VO_2$max (ml/kg/min) | 16.0 ±0.6 | (12.9-18.0) |
| Exercise Tolerance $VO_2$ max (kp) | 2.95 ±0.2 | (2.0-3.5) |
| Exercise Tolerance 50% $VO_2$ max (kp) | 1.1 ±0.1 | (1.0-1.25) |
| Exercise Tolerance $VO_2$ max (watts) | 177.0 ±9.9 | (120-210) |
| Exercise Tolerance 50% $VO_2$ max (watts) | 66.0 ±3.5 | (60-75) |

(continued)

| Profile | Value |
|---|---|
| Fasting Blood Sugar (mg/dl) | 100.2 ±4.8 (81-132) |
| Fasting Insulin (μU/ml) | 9.8 ±2.7 (3.9-32.0) |
| HOMA-R | 2.5 ±0.8 (0.95-8.35) |
| Glycosylated Hemoglobin (%) | 5.4 ±0.2 (4.8-6.5) |
| Total Cholesterol (mg/dl) | 207 ±10.8 (146-254) |
| Neutral Fat (mg/dl) | 152 ±11.0 (94-212) |
| HDL Cholesterol (mg/dl) | 48.2 ±1.7 (42.9-59.0) |
| LDL Cholesterol (mg/dl) | 134 ±6.7 (111-166) |
| AST (IU/L) | 29.8 ±4.1 (17-52) |
| ALT (IU/L) | 44.5 ±9.2 (16-102) |
| γ-GTP (IU/L) | 54.4 ±11.8 (20-118) |
| Systolic Blood Pressure (mmHg) | 116 ±5.1 (94-142) |
| Diastolic Blood Pressure (mmHg) | 77 ±4.1 (60-96) |
| Values are shown as the means ± SEM of 10 subjects. | |

Here, ALT, AST and γ-GTP are aspartate aminotransferase, alanine aminotransferase and γ-glutamyl transpeptidase, respectively. HOMA-R (Homeostasis Model Assessment insulin resistance index) is an index representing insulin resistance and represented by the following formula:

$$\text{HOMA-R} = \text{Fasting Blood Sugar (mg/dl)} \times \text{Fasting Blood Insulin Level } (\mu\text{U/ml}) \div 405$$

The method of physical measurement for the subjects was based on ACSM guideline (American College of Sports Medicine, 2001). Respiratory gas was collected and analyzed by a respiratory gas automatic analyzer (Oxycon Alpha, manufactured by Mijnhardt) every 30 seconds during the test. Maximal oxygen uptake ($VO_2$max) was calculated by increasing exercise loading gradually with a bicycle ergometer (818E, manufactured by Monark).

The tests were conducted as a double-blind crossover tests and dietary restriction was imposed on the subjects from two days before the tests. All subjects were instructed to abstain from alcohol, caffeine, green tea and high-intensity exercising 24 hours before the tests. Moreover, they recorded food intake two days before the first tests and repeated the same food intake before the second tests. This is for standardizing of the food intake before the tests and for minimizing the change of glycogen storage. All subjects took the same food as the dinner a day before all tests (725kcal, 60% of the energy from carbohydrate, 30% from lipid and 10% from protein). After an overnight (10 hours to 12 hours) fast, the tests were conducted. During the fasting period, they were permitted to take water freely.

In order to avoid the effect of circadian rhythm, the tests were started at the same time. After the 15 minutes rest, the first venous blood sampling was conducted. The subjects exercised for an hour with loading of about 50% of $VO_2$ max by a bicycle ergometer. After exercising, blood sampling was conducted and they took the test drinks for 5 minutes. Blood sampling was conducted at time points of 10, 20, 30, 45, 60, 75, 90, 105 and 120 minutes after the test drink was taken in. The collected blood samples were submitted for various biochemical measurements. After the drink intake, their heart rates were continuously measured with an electrocardiograph (Dyan Scope, manufactured by Fukuda Denshi).

On the assumption that oxidation of proteins during exercising can be ignored, oxidation of total carbohydrates and lipids was calculated from $VO_2$ and $VCO_2$ using Frayn's equation. The $VO_2$ and $VCO_2$ represent oxygen intake and carbon dioxide excretion in L/min, respectively. Respiratory quotient (RQ) was calculated from oxygen intake and carbon dioxide excretion. Calorie consumption rate was calculated from RQ and $VO_2$ using Lusk's equation.

Figure 1 shows blood glucose levels changes over time by drink intake after exercising. As shown in Figure 1, in taking sucralose, a blood sugar level did not rise since no sugar was taken. In taking high-fructose corn syrup, a blood sugar level rose immediately thereafter and started to drop 45 minutes thereafter. In taking palatinose, a blood sugar level rose slowly thereafter, surpassed that in the case of taking high-fructose corn syrup around 60 minutes thereafter, and continued maintaining a relatively high value.

Figure 2 shows blood insulin levels changes over time by drink intake after exercising. It was confirmed that insulin secretion was mild in taking palatinose while it was stimulated in taking high-fructose corn syrup. Figure 3 shows blood C-peptide levels changes over time by drink intake after exercising. Since C-peptide is an index of insulin secretion, Figure 3 shows a tendency similar to Figure 2.

Figure 4 shows blood free fatty acid levels changes over time by drink intake after exercising. Though free fatty acid levels rose after exercising as shown in Figure 4 due to promoted metabolism of lipid by exercising, in taking high-fructose corn syrup, a free fatty acid level dropped 30 minutes thereafter. This is thought to result from a switch to metabolism of taken sugar. In taking sucralose, the higher values were maintained. In taking palatinose, the values were shown between those in taking high-fructose corn syrup and in taking sucralose.

Figure 5 shows blood lactic acid levels changes over time by drink intake after exercising. It was found that high-fructose corn syrup entered in glycolytic metabolism after its intake since a lactic acid level rose in taking it. On the other hand, in taking sucralose, there was no change. In taking palatinose, the values were shown between those in taking high-fructose corn syrup and in taking sucralose.

The changes over time of respiratory quotient, the amount of oxidized carbohydrate and the amount of oxidized lipids provided by drink intake after exercising are shown in Figure 6, 7 and 8, respectively. The respiratory quotient approximates to 1.0 when carbohydrates are oxidized, while the respiratory quotient approximates to 0.7 when lipids are oxidized. As shown in Figure 6, in taking sucralose, the respiratory quotient close to 0.7 was maintained which shows that lipids were metabolized. In taking high-fructose corn syrup, it was found that the metabolism of lipids was replaced by the metabolism of carbohydrates since the respiratory quotient rose to the value close to 1.0. In taking palatinose, the values were shown between those in taking high-fructose corn syrup and in taking sucralose. As shown in Figure 7, the amount of oxidized carbohydrate showed higher values in taking high-fructose corn syrup, while showed lower values in taking sucralose. In taking palatinose, the values were shown between those of high-fructose corn syrup and sucralose. The amount of oxidized lipids in Figure 8 showed lower values in taking high-fructose corn syrup while higher values in taking sucralose, contrary to the results for the amount of oxidized carbohydrate. In taking palatinose, the values were shown between those of high-fructose corn syrup and sucralose. It is recognized from Figure 4, 5, 7 and 8 that lipid oxidation is rather difficult to prevent in taking palatinose while lipid oxidation is maintained in taking sucralose and it is replaced by carbohydrate oxidation in taking high-fructose corn syrup.

Figure 9 to Figure 11 show changes over time of blood neutral fat levels, blood epinephrine levels and blood nolepinephrine levels by drink intake after exercising. Epinephrine and nolepinephrine have an effect of promoting fat metabolism. In neutral fat, epinephrine and nolepinephrine, there were no differences between each drink. It was found that taking high-fructose corn syrup or palatinose had no effect on the metabolic pathway in which neutral fat, epinephrine and nolepinephrine participated.

Figure 12 and Figure 13 show changes over time of blood 3-OHBA levels and blood total ketone bodies levels by drink intake after exercising. In taking sucralose, 3-OHBA and blood total ketone bodies rose together. This is because lipid oxidation is facilitated. On the other hand, in taking high-fructose corn syrup, 3-OHBA and blood total ketone bodies dropped. Surprisingly, it was found that in taking palatinose, ketone bodies dropped without their accumulation in blood although lipids ware oxidized (Figure 8).

The above-mentioned facts revealed that in diabetic patients, patients with metabolic syndromes, and obese persons who are subject to a limited intake of carbohydrate, taking palatinose after exercising could maintain lipid metabolism and sufficient exercising effects as well as inhibit accumulation of ketone bodies. Therefore, it was shown that taking palatinose after exercising could prevent and improve ketosis or acidosis.

Example 2: Sugar stick containing palatinose

Palatinose and sucrose having the same weight were mixed and filled in stick packs so that 3.5 g of palatinose and sucrose would be contained per pack, respectively.

Example 3: Gum syrup containing palatinose

With the formulation shown in Table 2 below, gum syrup containing palatinose was produced. The production was conducted by putting palatinose and gum arabic together, conducting powder mixing, adding high-fructose corn syrup and water thereto, boiling and mixing. The solution above was adjusted to 30 by a Ref Brix scale.

[Table 2]

| Ingredient | g |
|---|---|
| Crystalline palatinose (Crystalline Palatinose-IC, trade name, manufactured by Mitsui Sugar Co., Ltd.) | 27.0 |
| high-fructose corn syrup (EP-O, trade name, San-ei Sucrochemical Co., Ltd., 75%) | 48.0 |
| Gum arabic | 4.0 |
| Water | 200.0 |

Example 4: Tablet containing palatinose

With the formulation shown in Table 3 below, a tablet containing palatinose was produced. The production was conducted by applying 300 kg/cm$^3$ of tabletting pressure to the mixed powder with the formulation shown below to form a tablet with 18mm of diameter, 5mm of thickness and 1.5 g of weight.

[Table 3]

| Ingredient | Combination ratio (weight) |
|---|---|
| Powdered palatinose (Paltinose Powder-ICP, trade name, manufactured by Mitsui Sugar Co., Ltd.) | 27.5 |
| Powdered sugar | 27.5 |
| Citric acid | 1 |
| Sugar ester | 1 |
| Aspartame | 0.05 |
| Vitamin P | 0.0002 |
| Water | 0.6 |
| Lemon juice | Proper quantity |

Example 5: Powder drink containing palatinose

With the formulation shown in Table 4 below, a powder drink containing palatinose was produced using a universal mixer according to a normal method. 50 g to 60 g of the obtained powder drink was dissolved in 250 ml to 1000 ml of water to prepare a refreshing drink.

[Table 4]

| Ingredient | Combination ratio (weight) |
|---|---|
| Crystalline palatinose (Crystalline Palatinose-IC, trade name, manufactured by Mitsui Sugar Co., Ltd.) | 42.35 |
| Sugar (granulated sugar) | 42.35 |
| Powder juice | 10 |
| Anhydrous citric acid | 3 |
| Sodium citrate | 0.4 |
| L-ascorbic acid | 0.5 |
| Sodium ascorbate | 0.3 |
| Riboflavin (content of 10% by weight) | 0.1 |

Example 6: Refreshing drink (sports drink) containing palatinose

With the formulation shown in Table 5 below, a refreshing drink containing palatinose was produced. The production was conducted by dissolving the materials below in 250 ml of boiling water, followed by filling the resultant solution into a beverage can (for 250 ml).

[Table 5]

| Ingredient | g/can |
|---|---|
| Crystalline palatinose (Crystalline Palatinose-IC, trade name, manufactured by Mitsui Sugar Co., Ltd.) | 4 |
| Sugar (granulated sugar) | 4 |
| Citric acid | 0.15 |
| Vitamin C | 0.03 |
| Sodium chloride | 0.05 |
| Potassium chloride | 0.04 |
| Calcium chloride | 0.012 |
| Magnesium carbonate | 0.002 |
| Sodium glutamate | 0.006 |
| Stevia | 0.01 |
| Vitamin P | 0.0004 |
| Flavor | Proper quantity |

Example 7: Refreshing drink (sports drink) containing palatinose

With the formulation shown in Table 6 below, a refreshing drink containing palatinose was produced. The production was conducted by dissolving the materials below in 250 ml of boiling water, followed by filling the resultant solution into a beverage can (for 250 ml).

[Table 6]

| Ingredient | Combination ratio (weight) |
|---|---|
| Crystalline palatinose (Crystalline Palatinose-IC, trade name, manufactured by Mitsui Sugar Co., Ltd.) | 10 |
| Citric acid | 0.15 |
| Vitamin C | 0.03 |
| Sodium chloride | 0.05 |
| Potassium chloride | 0.04 |
| Calcium lactate | 0.012 |
| Magnesium carbonate | 0.002 |
| Sodium glutamate | 0.006 |
| Flavor | Proper quantity |

Example 8: Refreshing drink (near water) containing palatinose

With the formulation shown in Table 7 below, a refreshing drink containing palatinose was produced. The production was conducted by dissolving the materials below in 500 ml of boiling water, followed by filling the resultant solution into a plastic bottle(for 500 ml).

[Table7]

| Ingredient | Combination ratio (weight) |
|---|---|
| Crystalline palatinose (Crystalline Palatinose-IC, trade name, manufactured by Mitsui Sugar Co., Ltd.) | 5 |
| Citric acid | 0.15 |
| Vitamin C | 0.03 |
| Sodium chloride | 0.05 |
| Potassium chloride | 0.04 |
| Calcium lactate | 0.012 |
| Magnesium carbonate | 0.002 |
| Sodium glutamate | 0.006 |
| Stevia | 0.005 |
| Vitamin P | 0.0002 |
| Flavor | Proper quantity |

Example 9: Powder drink containing palatinose

With the formulation shown in Table 8 below, a powder drink containing palatinose was produced using a universal mixer according to a normal method. 50 g to 60 g of the obtained powder drink was dissolved in 250 ml to 1000 ml of water to prepare a refreshing drink.

[Table 8]

| Ingredient | Combination ratio (weight) |
|---|---|
| Crystalline palatinose (Crystalline Palatinose-IC, trade name, manufactured by Mitsui Sugar Co., Ltd.) | 84.7 |
| Powder juice | 10.0 |
| Anhydrous citric acid | 3.0 |
| Sodium citrate | 0.4 |
| L-ascorbic acid | 0.5 |
| Sodium ascorbate | 0.3 |
| Riboflavin (content of 10% by weight) | 0.1 |
| Flavor | Proper quantity |

Example 10: Jelly drink containing palatinose

With the formulation shown in Table 9 below, a jelly drink containing palatinose (as a drink contained in a normal 180 g pouch) was produced.

[Table 9]

| Ingredient | Combination ratio (weight) |
|---|---|
| Crystalline palatinose (Crystalline Palatinose-IC, trade name, manufactured by Mitsui Sugar Co., Ltd.) | 15 |
| Dietary fiber | 5 |
| Gelling agent | 1 |
| Citric acid | 0.3 |
| Vitamin C | 0.06 |
| Sodium citrate | For pH adjustment |
| Flavor | Proper quantity |

The compounds shown in Examples 2 to 10 are ketone body accumulation inhibitors containing palatinose as an active ingredient.

## Claims

1. Isomaltulose for use in the treatment of ketosis caused by exercising in diabetic patients, patients with metabolic syndrome and obese persons.

## Patentansprüche

1. Isomaltulose zur Verwendung bei der Behandlung einer durch körperliche Bewegung verursachten Ketose bei diabetischen Patienten, Patienten mit metabolischem Syndrom und fettleibigen Personen.

## Revendications

1. Isomaltulose destiné à être utilisé dans le traitement de la cétose causée par l’exercice physique chez les patients diabétiques, les patients atteints d’un syndrome métabolique et les personnes obèses.

*Fig.1*

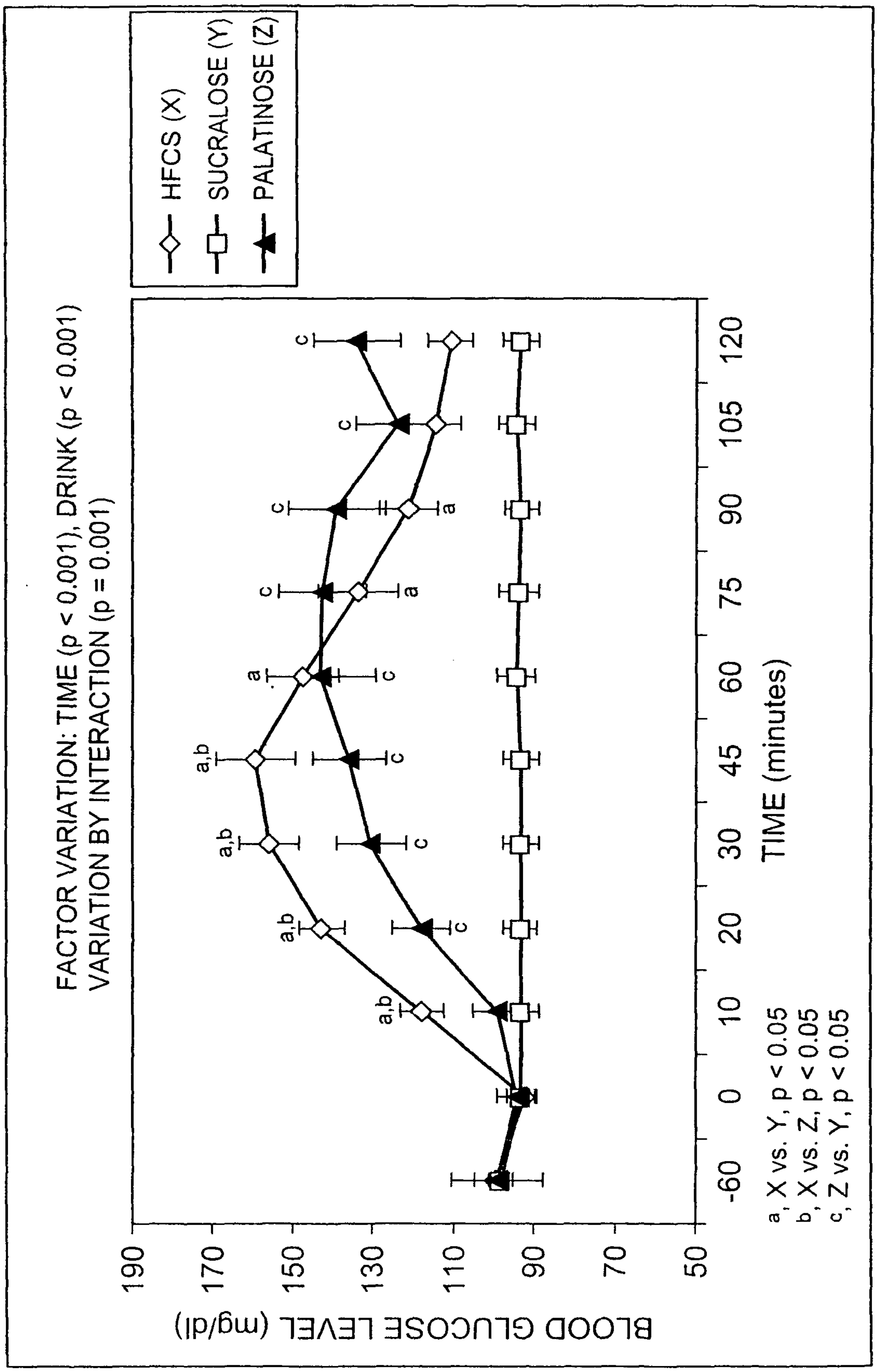
FACTOR VARIATION: TIME (p < 0.001), DRINK (p < 0.001)
VARIATION BY INTERACTION (p = 0.001)
HFCS (X)
SUCRALOSE (Y)
PALATINOSE (Z)
BLOOD GLUCOSE LEVEL (mg/dl)
190
170
150
130
110
90
70
50
-60
0
10
20
30
45
60
75
90
105
120
TIME (minutes)
a, X vs. Y, p < 0.05
b, X vs. Z, p < 0.05
c, Z vs. Y, p < 0.05
a,b
c
a

Fig.2

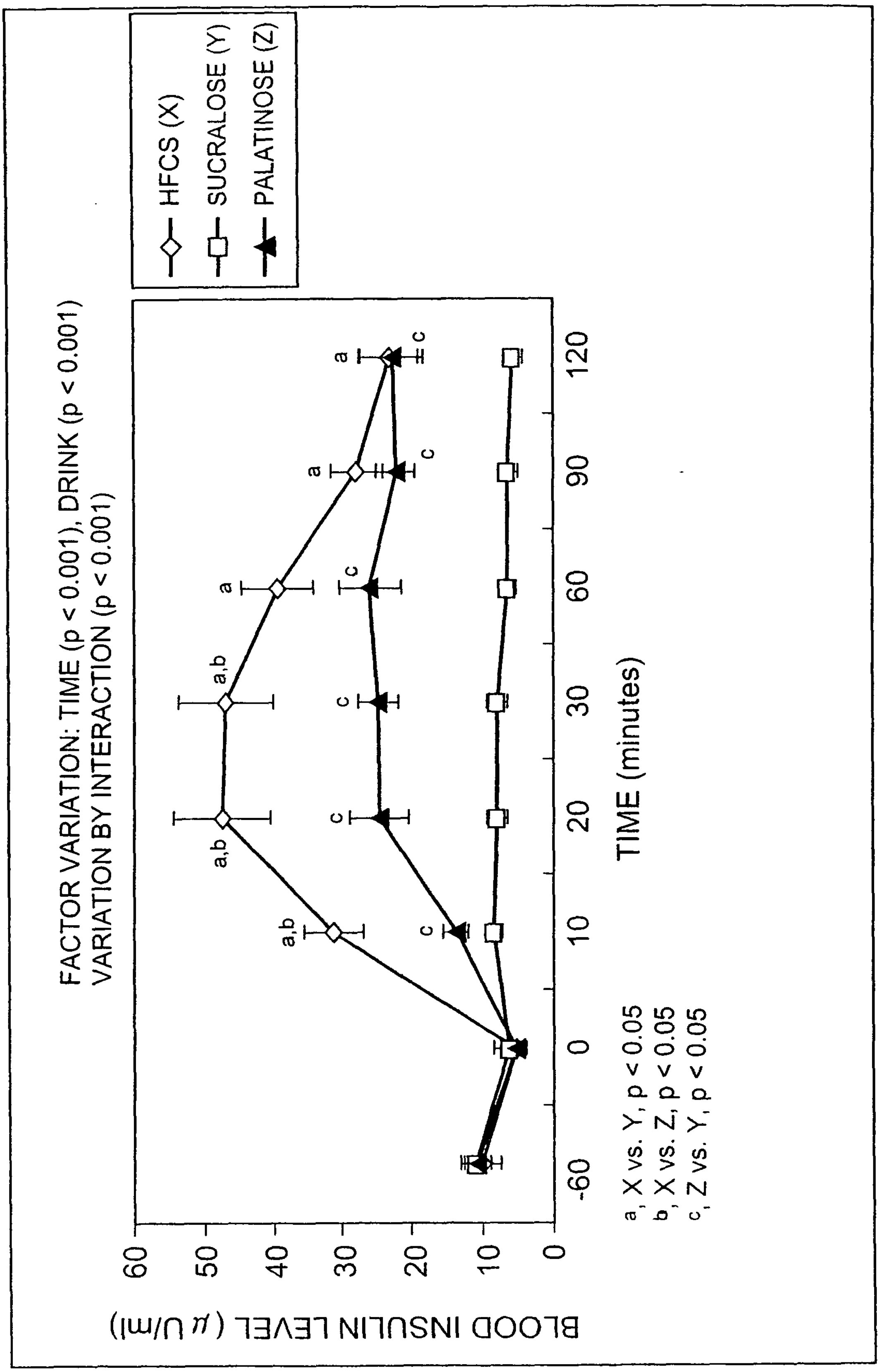
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
BLOOD INSULIN LEVEL (μU/ml)
60
50
40
30
20
10
0
-60
0
10
20
30
60
90
120
TIME (minutes)
a,b
a
c
a, X vs. Y, p < 0.05
b, X vs. Z, p < 0.05
c, Z vs. Y, p < 0.05

Fig.3

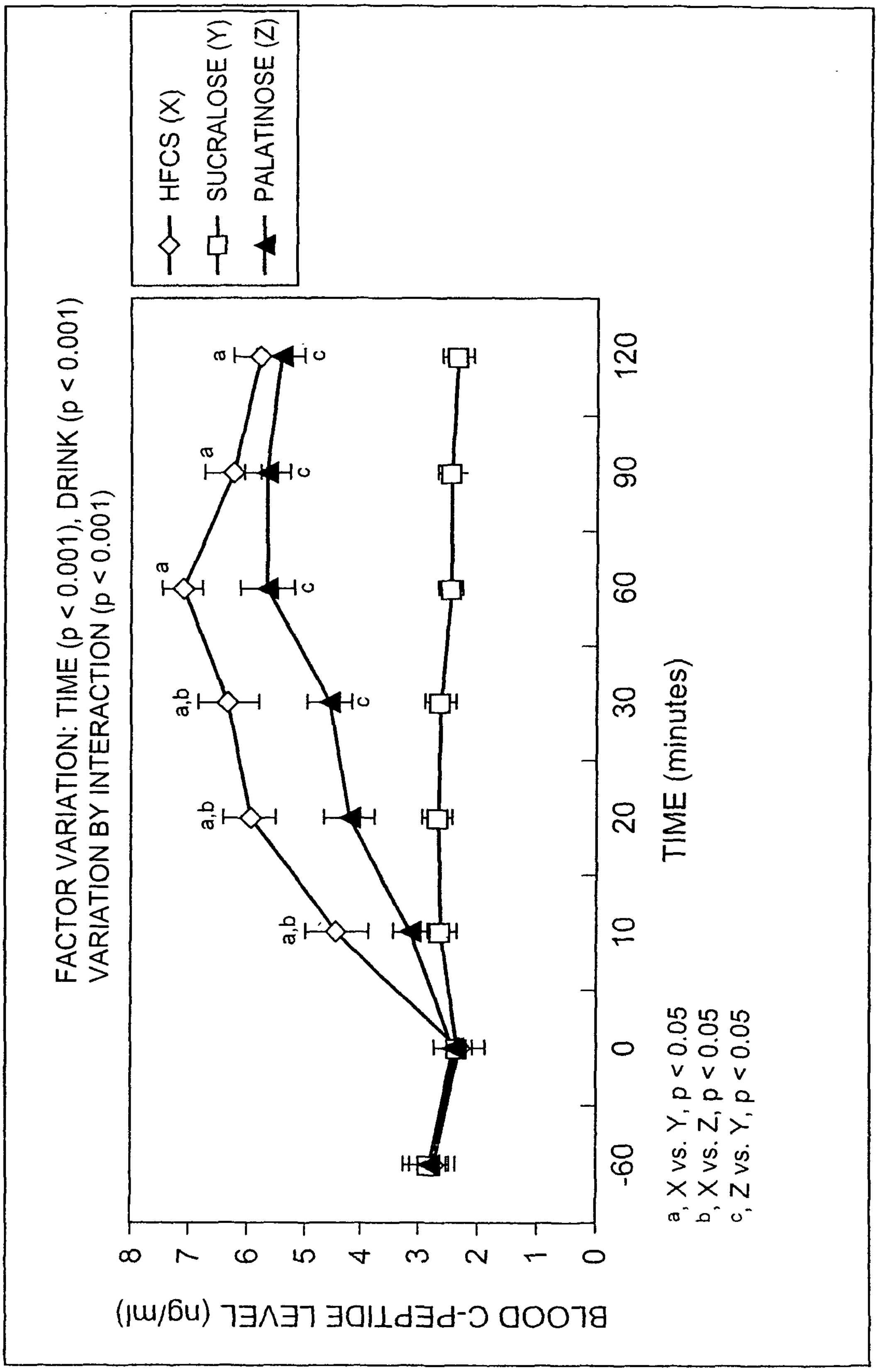
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
BLOOD C-PEPTIDE LEVEL (ng/ml)
TIME (minutes)
-60
0
10
20
30
60
90
120
0
1
2
3
4
5
6
7
8
a,b
a
c
a, X vs. Y, p < 0.05
b, X vs. Z, p < 0.05
c, Z vs. Y, p < 0.05

Fig.4

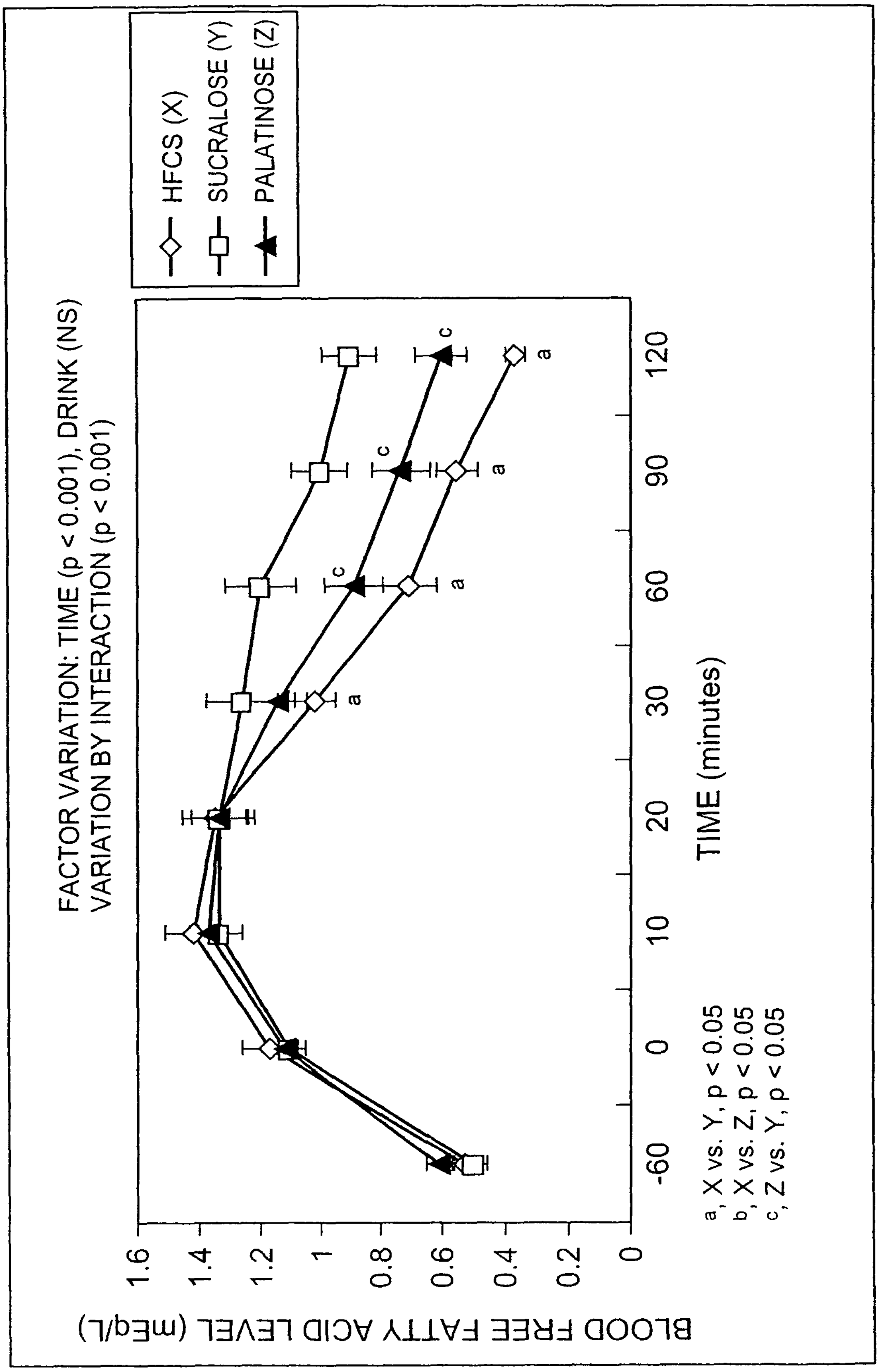
FACTOR VARIATION: TIME (p < 0.001), DRINK (NS)
VARIATION BY INTERACTION (p < 0.001)
HFCS (X)
SUCRALOSE (Y)
PALATINOSE (Z)
BLOOD FREE FATTY ACID LEVEL (mEq/L)
1.6
1.4
1.2
1
0.8
0.6
0.4
0.2
0
-60
0
10
20
30
60
90
120
TIME (minutes)
a
c
a, X vs. Y, p < 0.05
b, X vs. Z, p < 0.05
c, Z vs. Y, p < 0.05

*Fig.5*

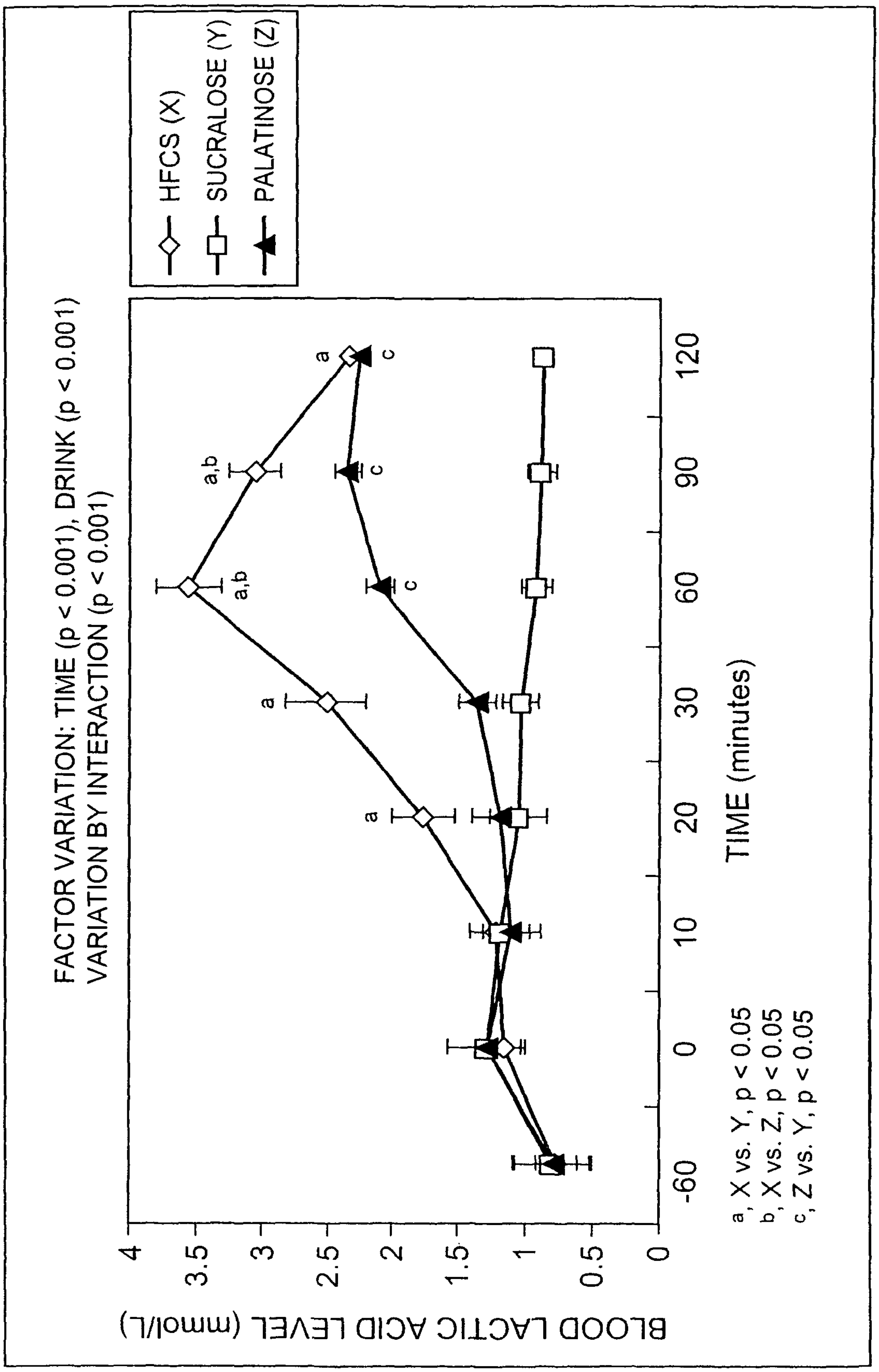
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
BLOOD LACTIC ACID LEVEL (mmol/L)
0
0.5
1
1.5
2
2.5
3
3.5
4
-60
0
10
20
30
60
90
120
TIME (minutes)
a
a,b
c
a, X vs. Y, p < 0.05
b, X vs. Z, p < 0.05
c, Z vs. Y, p < 0.05

Fig.6

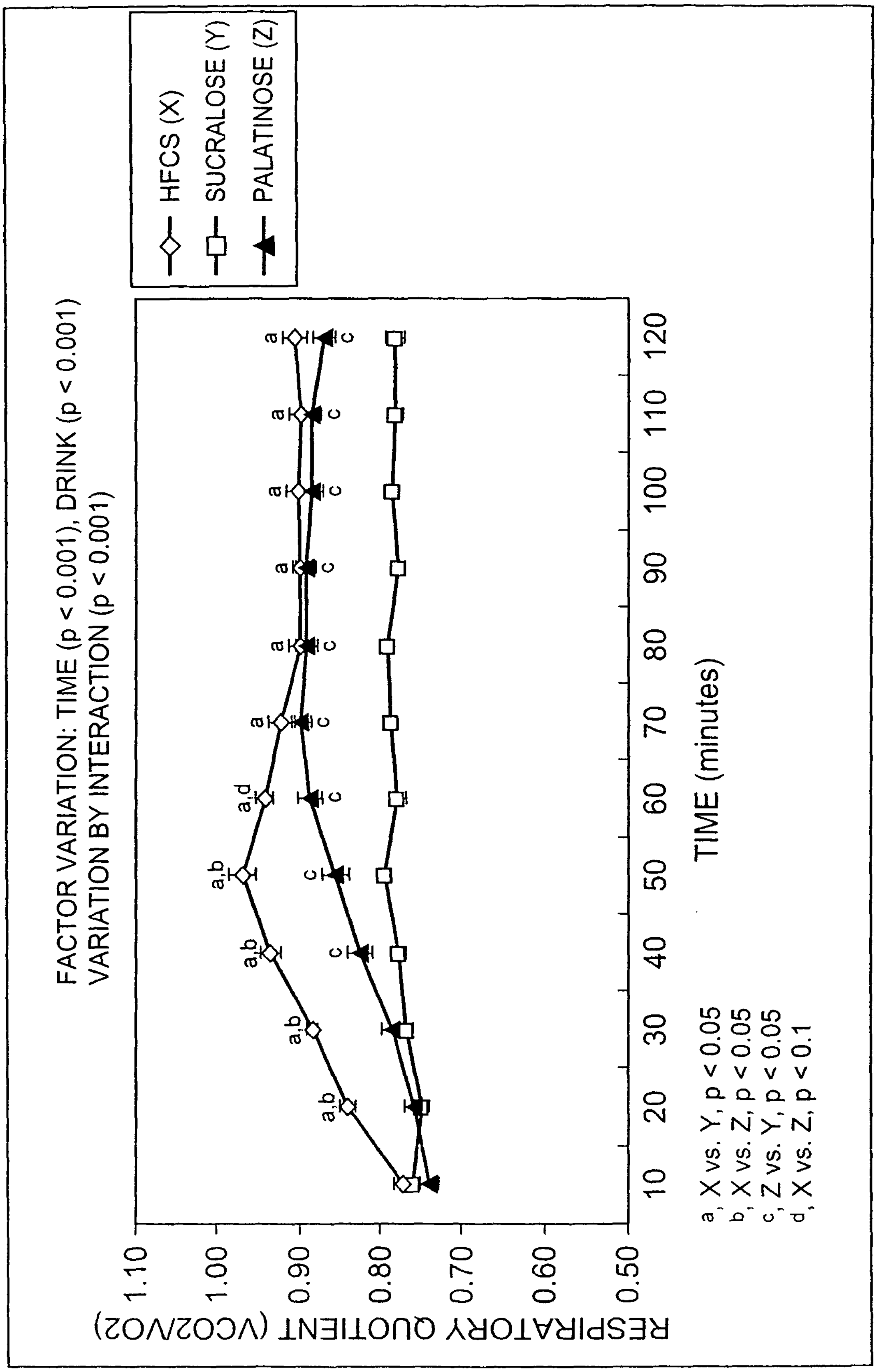
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
RESPIRATORY QUOTIENT (VCO2/VO2)
1.10
1.00
0.90
0.80
0.70
0.60
0.50
TIME (minutes)
10
20
30
40
50
60
70
80
90
100
110
120
a,b
a,d
a
c
a, X vs. Y, p < 0.05
b, X vs. Z, p < 0.05
c, Z vs. Y, p < 0.05
d, X vs. Z, p < 0.1

Fig.7

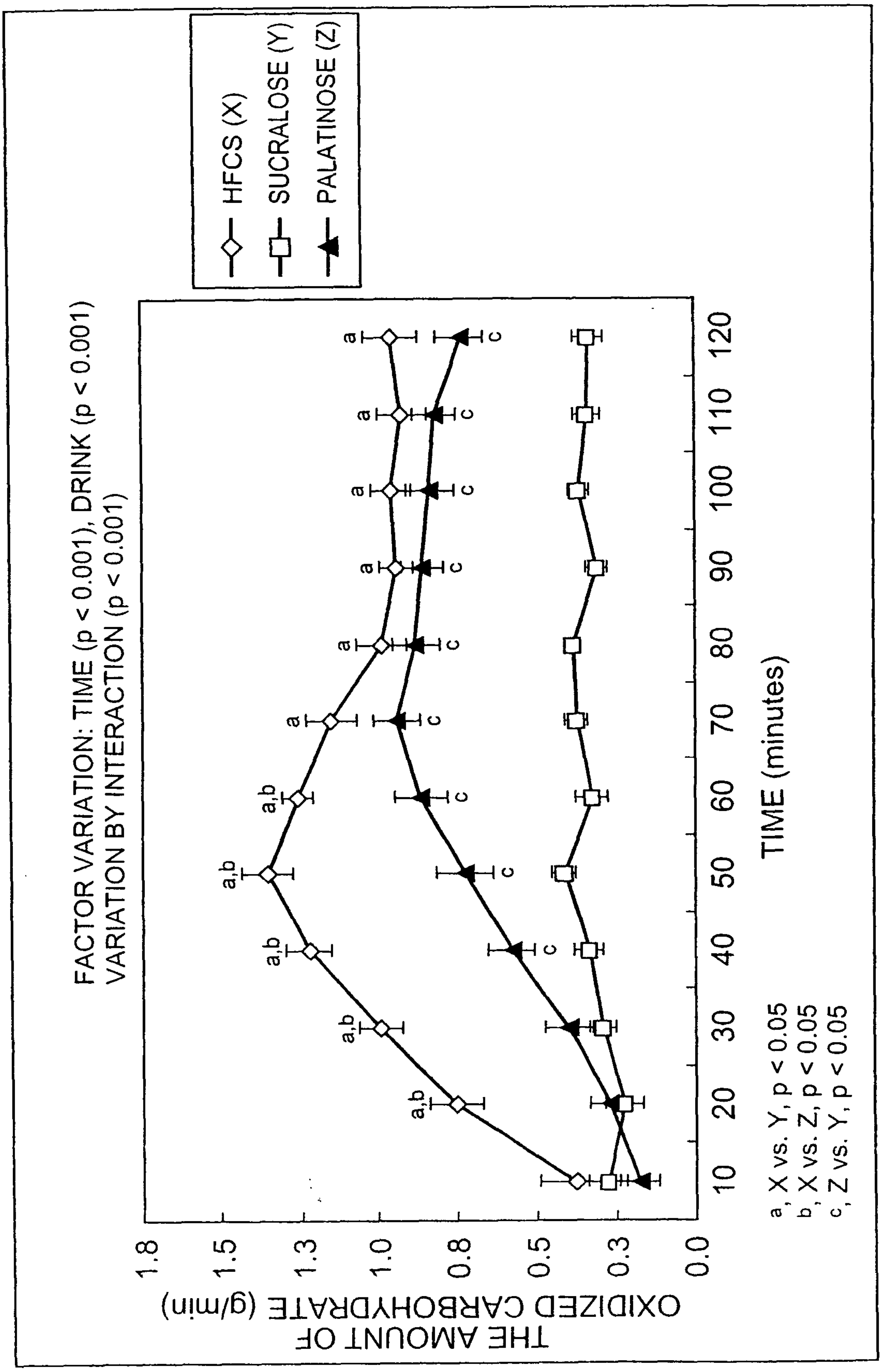
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
THE AMOUNT OF OXIDIZED CARBOHYDRATE (g/min)
1.8
1.5
1.3
1.0
0.8
0.5
0.3
0.0
10
20
30
40
50
60
70
80
90
100
110
120
TIME (minutes)
a,b
a
c
a, X vs. Y, p < 0.05
b, X vs. Z, p < 0.05
c, Z vs. Y, p < 0.05

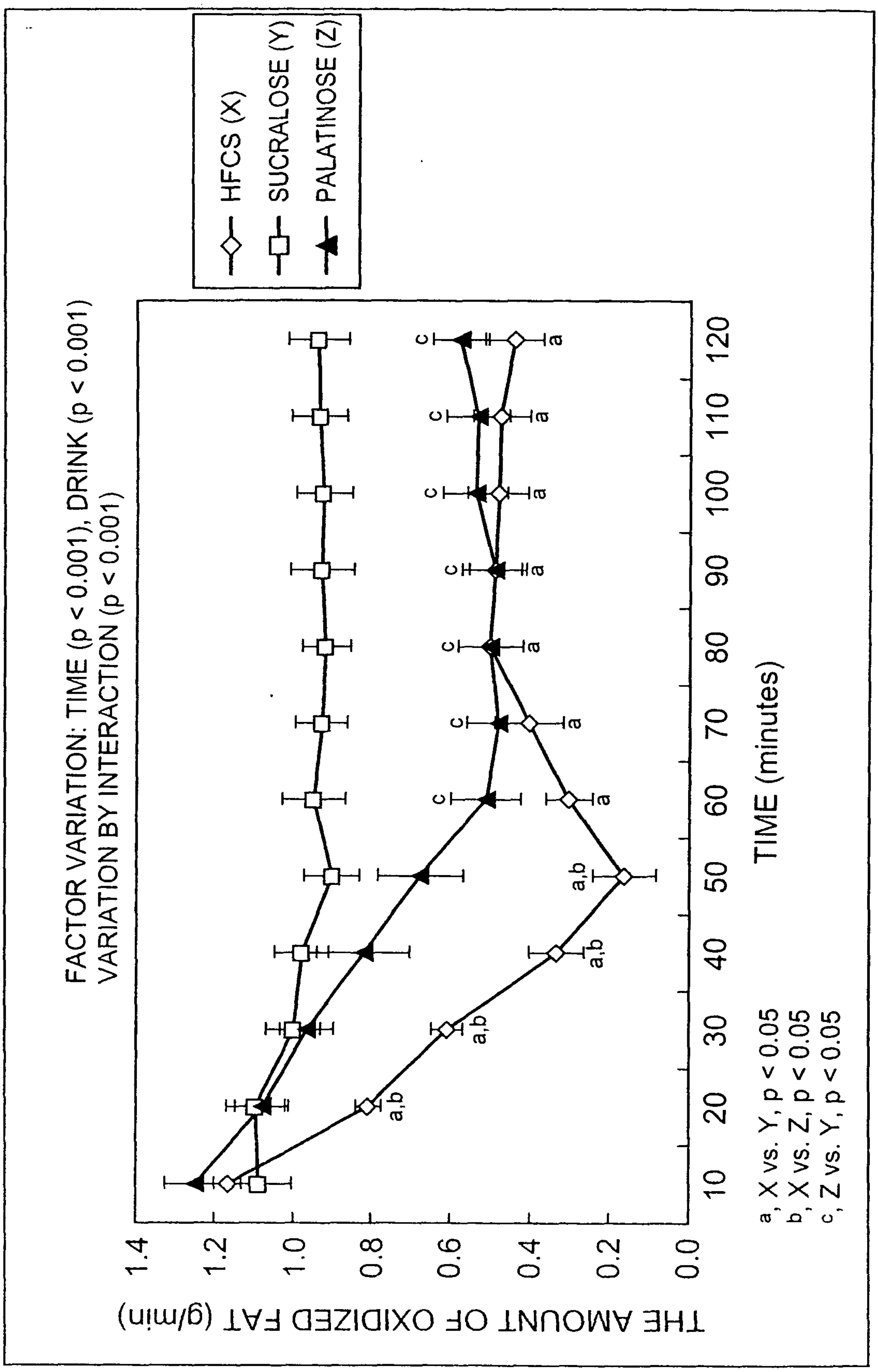
FACTOR VARIATION: TIME (p < 0.001), DRINK (p < 0.001)
VARIATION BY INTERACTION (p < 0.001)
HFCS (X)
SUCRALOSE (Y)
PALATINOSE (Z)
THE AMOUNT OF OXIDIZED FAT (g/min)
0.0
0.2
0.4
0.6
0.8
1.0
1.2
1.4
10
20
30
40
50
60
70
80
90
100
110
120
TIME (minutes)
a,b
c
a
a, X vs. Y, p < 0.05
b, X vs. Z, p < 0.05
c, Z vs. Y, p < 0.05

Fig.8

Fig.9

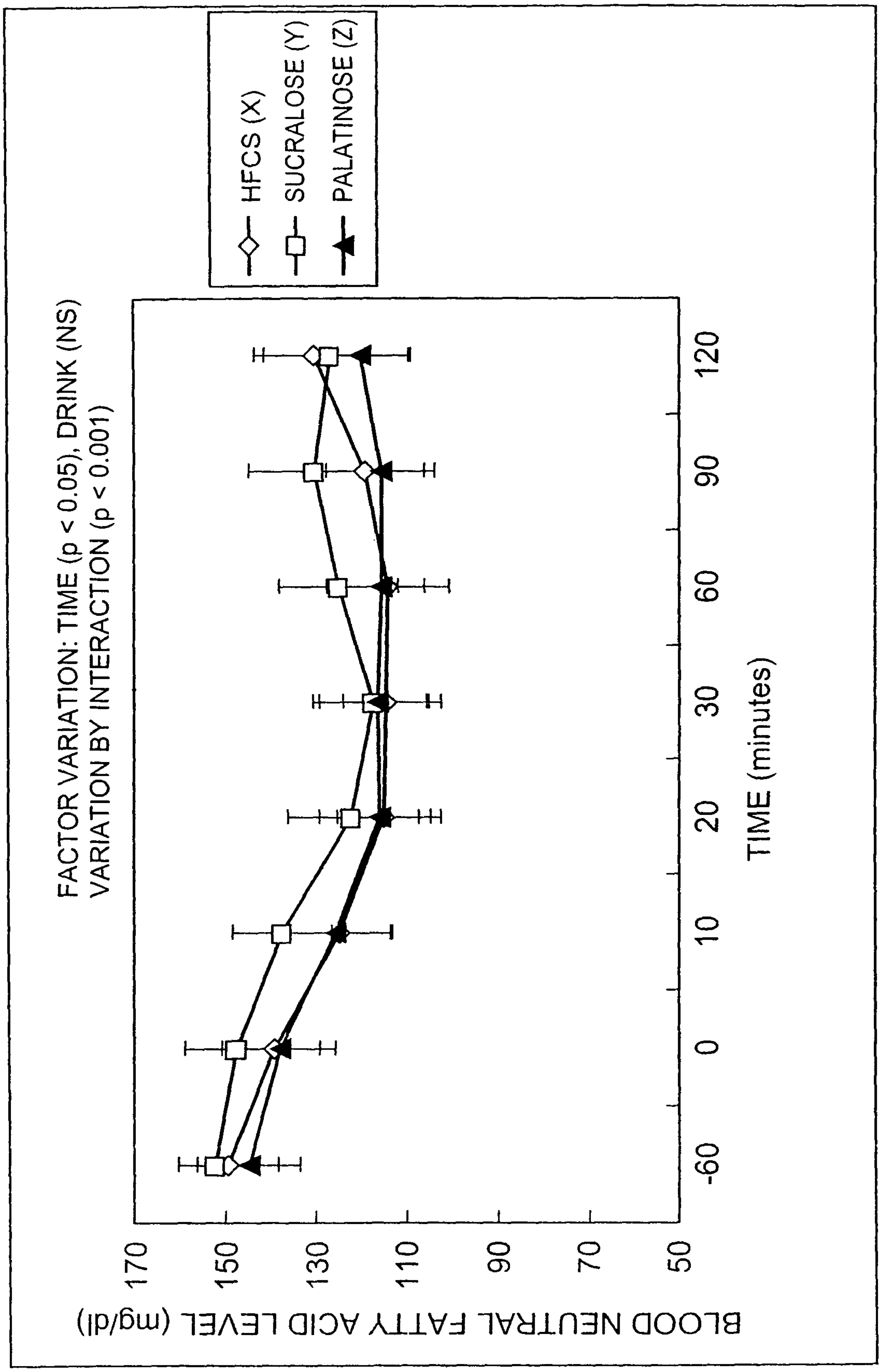
FACTOR VARIATION: TIME (p < 0.05), DRINK (NS)
VARIATION BY INTERACTION (p < 0.001)
HFCS (X)
SUCRALOSE (Y)
PALATINOSE (Z)
BLOOD NEUTRAL FATTY ACID LEVEL (mg/dl)
170
150
130
110
90
70
50
-60
0
10
20
30
60
90
120
TIME (minutes)

Fig.10

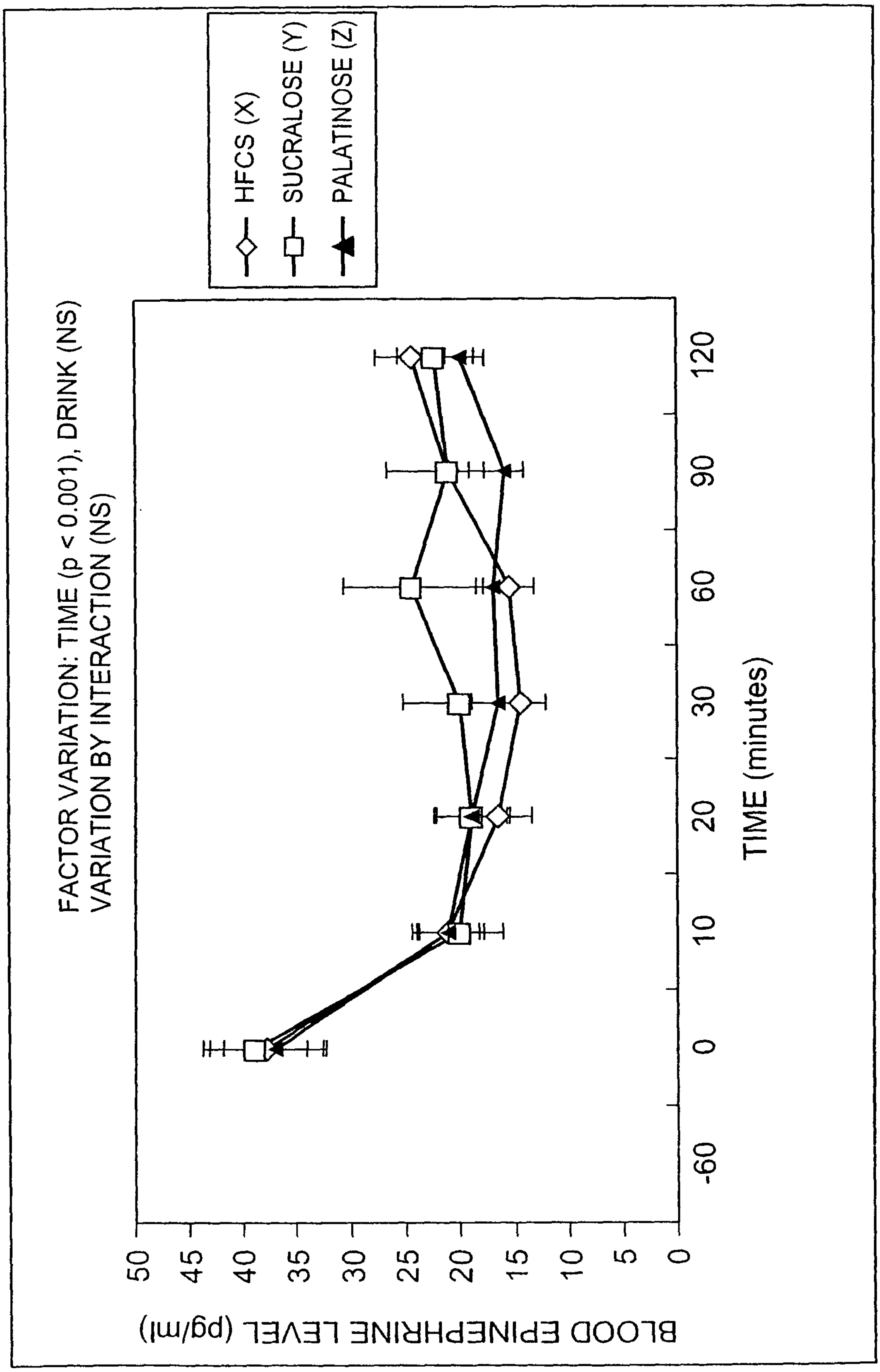
FACTOR VARIATION: TIME (p < 0.001), DRINK (NS)
VARIATION BY INTERACTION (NS)
HFCS (X)
SUCRALOSE (Y)
PALATINOSE (Z)
BLOOD EPINEPHRINE LEVEL (pg/ml)
0
5
10
15
20
25
30
35
40
45
50
TIME (minutes)
-60
0
10
20
30
60
90
120

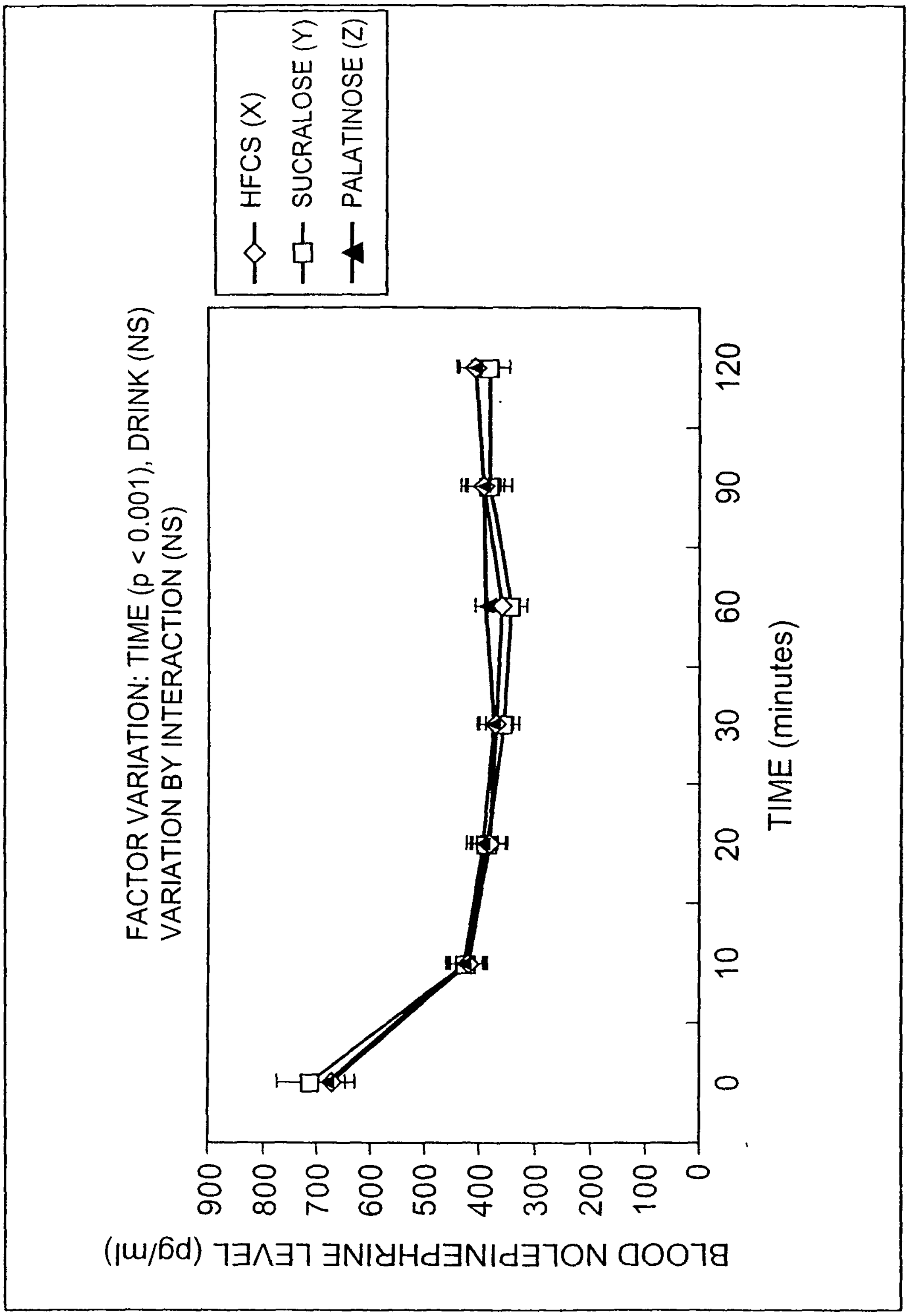
FACTOR VARIATION: TIME (p < 0.001), DRINK (NS)
VARIATION BY INTERACTION (NS)
HFCS (X)
SUCRALOSE (Y)
PALATINOSE (Z)
BLOOD NOLEPINEPHRINE LEVEL (pg/ml)
900
800
700
600
500
400
300
200
100
0
0
10
20
30
60
90
120
TIME (minutes)

Fig.11

Fig.12

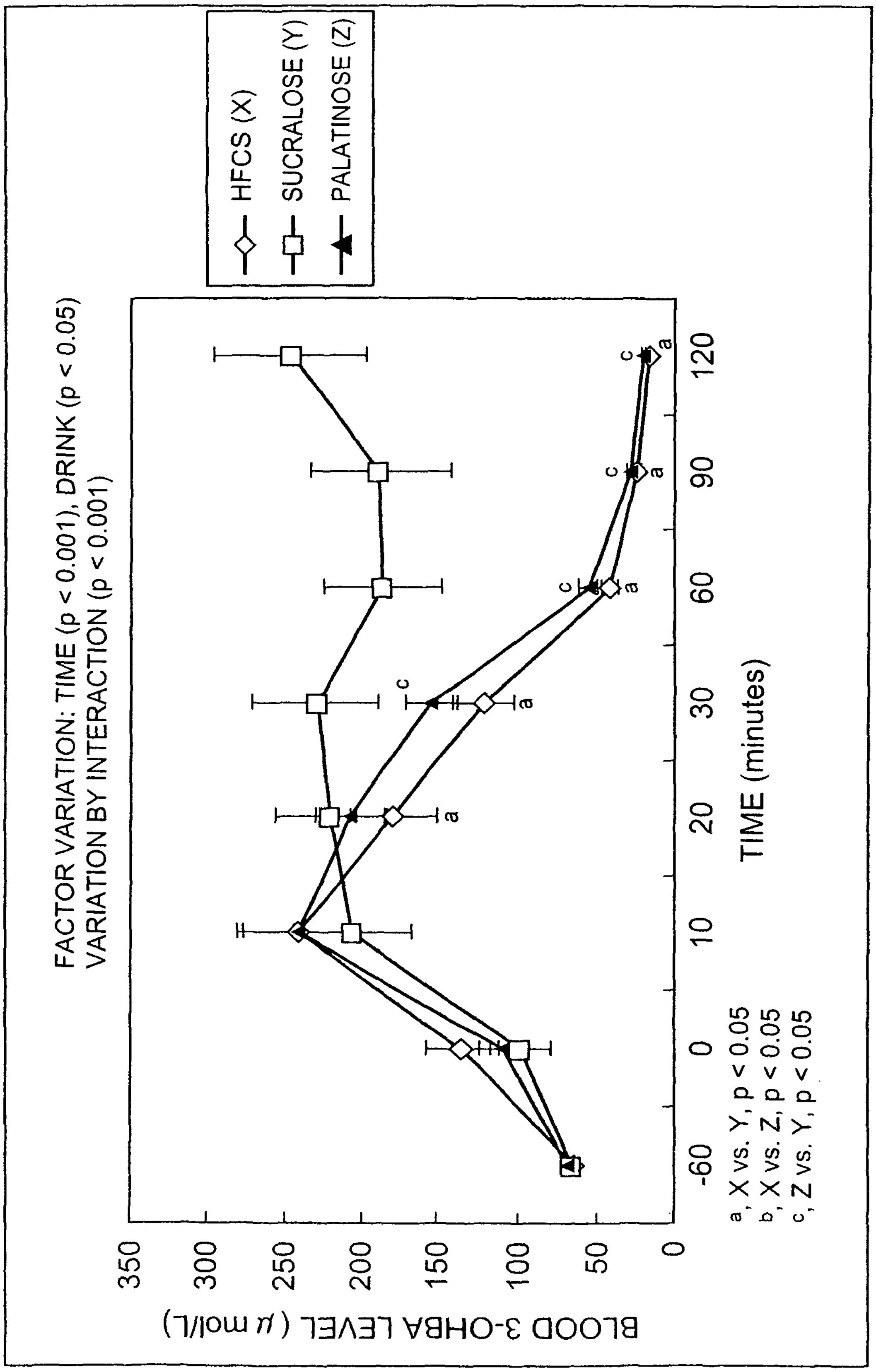
FACTOR VARIATION: TIME (p < 0.001), DRINK (p < 0.05)
VARIATION BY INTERACTION (p < 0.001)
BLOOD 3-OHBA LEVEL (μ mol/L)
350
300
250
200
150
100
50
0
-60
0
10
20
30
60
90
120
TIME (minutes)
HFCS (X)
SUCRALOSE (Y)
PALATINOSE (Z)
a
c
a, X vs. Y, p < 0.05
b, X vs. Z, p < 0.05
c, Z vs. Y, p < 0.05

Fig.13

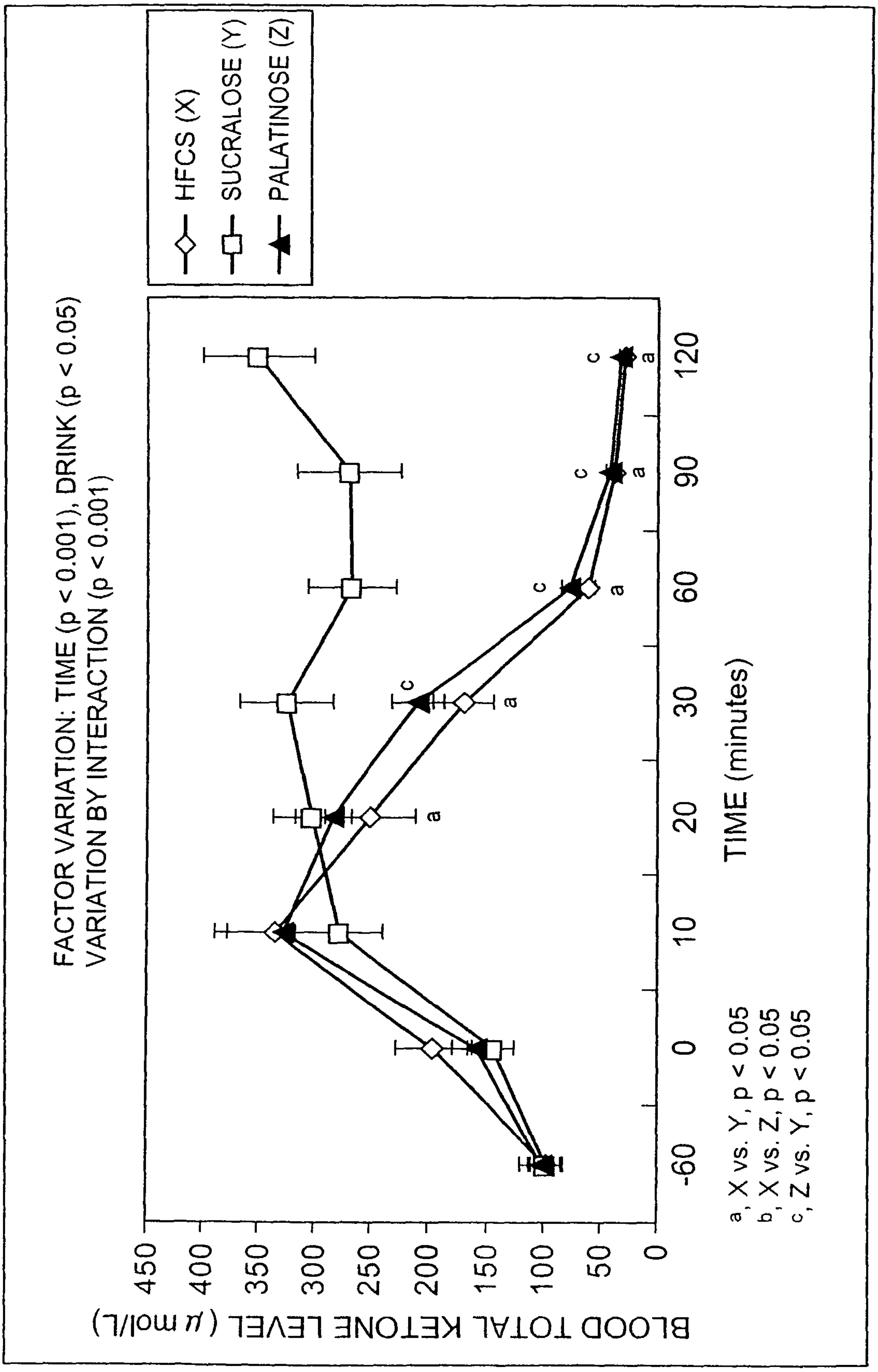
FACTOR VARIATION: TIME (p < 0.001), DRINK (p < 0.05)
VARIATION BY INTERACTION (p < 0.001)
HFCS (X)
SUCRALOSE (Y)
PALATINOSE (Z)
BLOOD TOTAL KETONE LEVEL (μ mol/L)
450
400
350
300
250
200
150
100
50
0
-60
0
10
20
30
60
90
120
TIME (minutes)
a
c
a, X vs. Y, p < 0.05
b, X vs. Z, p < 0.05
c, Z vs. Y, p < 0.05

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8026988 A

### Non-patent literature cited in the description

- *Examination and Technology,* 2004, vol. 32 (3), 276-277
- *Japanese Journal of Biomechanics in Sports and Exercise,* 1997, vol. 1 (1), 105-119
- *Nutrition Reviews,* 2005, vol. 63 (5), 133-157